# EUROPEAN PATENT APPLICATION

(11) **EP 2 218 432 A2**
(43) Date of publication of application: **18.08.2010**
(21) Application number: 10163999.5
(22) Date of filing: 20.02.2002
(51) Int. Cl.: A61J 1/00, C12M 1/00

(54) **Container for biological fluid**

(30) Priority: 20.02.2001 US 269383 P
(62) Divisional of application: 02742487.8
(71) Applicant: Pall Corporation, Port Washington, NY 11050 (US)
(72) Inventor: Donart, Michael, Simi Valley, CA 93065 (US)
(74) Representative: Hoeger, Stellrecht & Partner Patentanwälte

(57) **Abstract**

Containers and conduits comprising a copolymcr comprising cthylcnc and an acrylate arc disclosed.

## Description

### CROSS-REFERENCE TO RELATED PATENT APPLICATIONS

This patent application claims the benefit of U.S. Provisional Patent Application No. 60/269,383, filed February 20, 2001.

### FIELD OF THE INVENTION

This invention relates to containers and conduits for use with fluids, more preferably, for use with biological fluids such as blood and blood components.

### BACKGROUND OF THE INVENTION

Flexible polyvinyl chloride (PVC) bags are conventionally used for the collection and storage of blood and blood products. The PVC includes a plasticizer such as di (2-ethylhexyl) phthalate (DEHP) to provide flexibility. However, some plasticizers, e.g., DEHP, and n-butryl tri-n-hexyl citrate (BTHC), leach from the walls of the bags and into the blood and blood components during storage. Concerns have been raised over the potentially harmful effects of DEHP in blood products transfused into patients. Additionally, some plasticizers may adversely affect the blood components, e.g., inhibiting the coagulation ability of platelets. It has also been reported that the viability of platelets stored with a protein-free platelet additive solution in some plasticized bags is decreased after a day or two of storage.

Accordingly, there is a need in the art for a container that is suitable for storing blood or blood components, particularly blood or blood components mixed with protein-free additive solutions, for several days or more. There is also a need for such a container that exhibits little or no leaching of plasticizer into the blood or blood components.

The present invention provides for ameliorating at least some of the disadvantages of the prior art. These and other advantages of the present invention will be apparent from the description as set forth below.

### BRIEF SUMMARY OF THE INVENTION

In accordance with an embodiment of the invention, containers and/or conduits for use with a biological fluid are provided wherein the containers and/or conduits comprise a copolymer comprising ethylene and an acrylate, typically, ethylene and at least about 20 wt. % alkyl acrylate. In more preferred embodiments, the copolymer comprises ethylene and butyl acrylate, or ethylene and methyl acrylate.

Containers according to the invention are especially useful in closed systems, e.g., for storing a biological fluid such as a platelet-containing fluid, more preferably a platelet-containing plasma-depleted fluid mixed with a platelet additive fluid, for 5 days, or more. In preferred embodiments, the walls of the containers allow suitable gas transmission, especially oxygen transmission into the interior of the container, and this transmission can be desirable for various blood component metabolic functions during the storage period.

In accordance with an embodiment of a method according to the invention, a biological fluid, preferably a platelet-containing fluid, more preferably a platelet-containing plasma-depleted fluid mixed with a platelet additive fluid, and the fluid, e.g., a platelet- and additive- containing fluid, is stored, for a desired period of time, in a container having side walls manufactured from a polymeric film comprising a copolymer comprising ethylene and an acrylate. Preferably, the fluid can be stored for at least 5 days, in some embodiments, at least 7 days.

The following definitions are used in accordance with the invention:

Biological Fluid. A biological fluid includes any treated or untreated fluid associated with living organisms, particularly blood, including whole blood, warm or cold blood, and stored or fresh blood; treated blood, such as blood diluted with at least one physiological solution, including but not limited to saline, nutrient, and/or anticoagulant solutions; blood components, such as platelet concentrate (PC), platelet-rich plasma (PRP), platelet-poor plasma (PPP), platelet-free plasma, plasma, fresh frozen plasma (FFP), components obtained from plasma, packed red cells (PRC), transition zone material or buffy coat (BC); blood products derived from blood or a blood component or derived from bone marrow; stem cells, red cells separated from plasma and resuspended in physiological fluid or a cryoprotective fluid; and platelets separated from plasma and resuspended in physiological fluid or a cryoprotective fluid. The biological fluid may have been treated to remove some of the leukocytes before being processed according to the invention. As used herein, blood product or biological fluid refers to the components described above, and to similar blood products or biological fluids obtained by other means and with similar properties.

A "unit" is the quantity of biological fluid from a donor or derived from one unit of whole blood. It may also refer to the quantity drawn during a single donation. Typically, the volume of a unit varies, the amount differing from patient to patient and from donation to donation. Multiple units of some blood components, particularly platelets and buffy coat, may be pooled or combined, typically by combining four or more units.

As used herein, the term "closed" refers to a system that allows the collection and processing (and, if desired, the manipulation, e.g., separation of portions, separation into components, filtration, storage, and preservation) of biological fluid, e.g., donor blood, blood samples, and/or blood components, without the need to compromise the integrity of the system. A closed system can be as originally made, or result from the connection of system components using what are known as "sterile docking" devices. Illustrative sterile docking devices are disclosed in U.S. Patent Nos. 4,507,119, 4,737,214, and 4,913,756.

### BRIEF DESCRIPTION OF THE DRAWINGS

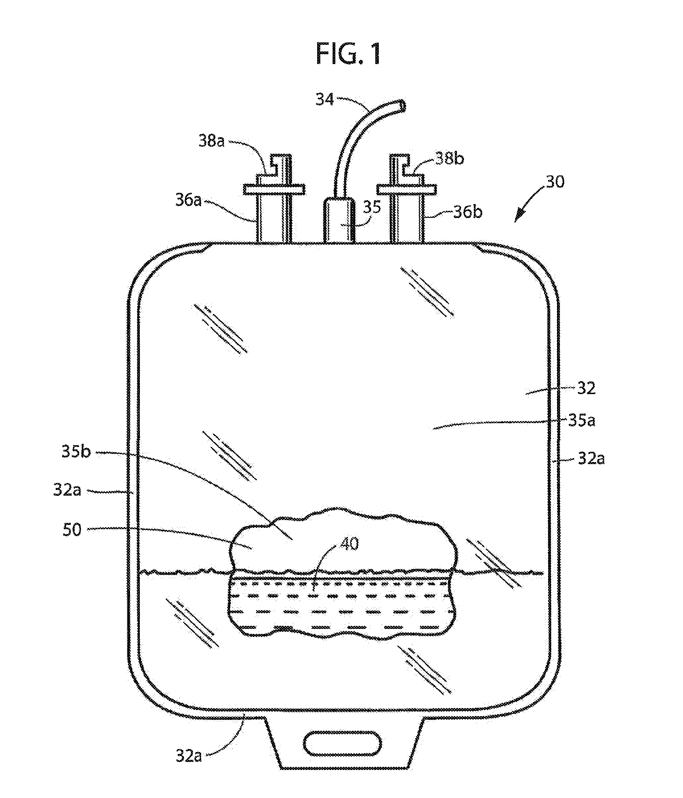

The Figure shows a partial cut-away plan view of an embodiment of a container produced in accordance with the present invention, wherein the container contains a biological fluid.

### DETAILED DESCRIPTION OF THE INVENTION

In accordance with an embodiment of the present invention, a biological fluid container is provided comprising a container having an internal volume, the container having first and second side walls, the walls comprising a polymeric film comprising at least one copolymer comprising ethylene and an acrylate. Preferably, the side walls comprise a polymeric film manufactured from a copolymer comprising ethylene and an alkyl acrylate, the alkyl acrylate comprising butyl acrylate or methyl acrylate.

A biological fluid container according to another embodiment of the invention comprises a container having an internal volume, the container having first and second side walls, the walls comprising a polymeric film manufactured from a copolymer comprising ethylene and at least about 20 weight percent alkyl acrylate.

In preferred embodiments of containers provided by the invention, the copolymer comprises ethylene and at least about 20 wt. % methyl acrylate or at least about 20 wt. % butyl acrylate.

The biological fluid container provided by the invention is especially suitable for storing a platelet-containing fluid, more preferably, for storing a platelet-containing plasma-depleted fluid mixed with a platelet additive solution, wherein the platelet- and additive-containing fluid has residual protein concentration (e.g., compared to the protein concentration of the non-plasma-depleted platelet-containing fluid) of about 35 % or less.

Typically, the polymeric film forming the side walls of the container has a 22° C room air oxygen transmission of about 12 µmoles or greater O₂/hr/350 cm² film surface area, preferably, a 22° C room air oxygen transmission of about 15 µmoles or greater O₂/hr/350 cm² film surface area, and even more preferably, a 22° C room air oxygen transmission of about 20 µmoles or greater O₂/hr/350 cm² film surface area.

An embodiment of a system for processing biological fluid according to the invention comprises at least two containers and at least one conduit in fluid communication with the two containers, wherein at least one container comprises a biological fluid container, the container having first and second side walls comprising a polymeric film manufactured from at least one copolymer comprising ethylene and an acrylate. In a preferred embodiment, the system comprises at least two flexible containers, each container having an internal volume and at least first and second ports capable of fluid communication with the internal volume, and at least one flexible hollow conduit, the conduit communicating with the first port of each container, wherein at least one container has first and second side walls comprising a polymeric film manufactured from a copolymer comprising ethylene and an alkyl acrylate, the alkyl acrylate comprising butyl acrylate or methyl acrylate. More preferably, the copolymer comprises ethylene and at least about 20 weight percent butyl acrylate or at least about 20 weight percent methyl acrylate, and in an even more preferred embodiment, the system comprises a closed system.

In accordance with another embodiment of the invention, a conduit for use with a biological fluid comprises a flexible hollow tubing comprising at least one copolymer comprising ethylene and an acrylate. In some embodiments of a system for processing biological fluid according to the invention, the system includes at least one flexible hollow conduit comprising a resin manufactured from at least one copolymer comprising ethylene and an acrylate. In preferred embodiments, the copolymer comprising ethylene and an acrylate comprises ethylene and an alkyl acrylate, more preferably wherein the alkyl acrylate is butyl acrylate or methyl acrylate.

An embodiment of a method according to the invention comprises passing a biological fluid into a container having first and second side walls, the walls comprising a polymeric film comprising a copolymer comprising ethylene and an acrylate. Typically, the method includes mixing the biological fluid with a biological fluid additive solution to provide a biological fluid additive solution mixture, and storing the mixture in the container for a desired period of time.

Another embodiment of a method for processing a biological fluid according to the invention comprises obtaining a platelet-containing biological fluid, mixing the platelet-containing biological fluid with a platelet-additive solution to provide a platelet-containing platelet additive mixture, and storing the mixture in a container having first and second side walls, the walls comprising a polymeric film manufactured from a copolymer comprising ethylene and an alkyl acrylate, the alkyl acrylate comprising butyl acrylate or methyl acrylate. In some embodiments, the method includes obtaining a platelet-containing biological fluid, depleting plasma from the platelet-containing biological fluid to provide a plasma-depleted platelet-containing fluid, mixing the plasma-depleted platelet-containing fluid with the platelet-additive solution to provide the platelet-containing platelet additive mixture, wherein the residual protein concentration in the mixture is about 35% or less of the protein concentration in the non-plasma-depleted platelet-containing biological fluid, and storing the mixture in the container.

Embodiments of the method can include pooling two or more volumes of plasma-depleted platelet-containing fluid, and mixing the pooled plasma-depleted platelet-containing fluid with the platelet-additive solution to provide a platelet-containing platelet additive mixture. For example, in one preferred embodiment, a plurality of units of whole blood are each processed to provide sedimented red cells, buffy coat, and platelet-poor-plasma, and the components are separated. The buffy coats (the plasma-depleted platelet-containing fluid) from each unit of whole blood are pooled, mixed with a protein-free platelet additive solution, and further processed to provide platelet concentrate in additive solution (a platelet- and additive-containing solution), that is stored, for a desired period of time, in a container having first and second side walls, the walls comprising a polymeric film manufactured from a copolymer comprising ethylene and an alkyl acrylate, the alkyl acrylate comprising butyl acrylate or methyl acrylate. Preferably, the residual protein concentration in the platelet concentrate in additive solution in the container is about 35% or less of the protein concentration in the non-plasma-depleted platelet-containing biological fluid

A typical embodiment of the method further comprises storing a biological fluid mixed with additive solution in the container for at least 2 days, preferably, at least 5 days, and in some embodiments, at least 7 days. If desired, a preferred embodiment of the method further comprises administering platelets to a patient.

Each of the components of the invention will now be described in more detail below, wherein like components have like reference numbers.

The Figure illustrates a container 30 partially cut away to show the liquid contents 40 (preferably a plasma-depleted platelet- and additive-containing fluid) in the interior volume 50 of the container. The illustrated container (also referred to as the bag) comprises a polymeric film 32 comprising a copolymer comprising ethylene and an acrylate, and the bag is sealed, e.g., the polymeric film is edge-sealed at 32a. The Figure also shows a conduit 34 in fluid communication with the container, and the container further comprises a port 35, and access ports 36a, 36b, wherein the access ports 36a and 36b can be accessed after manipulating caps 38a and 38b.

In this illustrated embodiment, the polymeric film 32 provides first side wall 35a and second side wall 35b of the container 30. The first and second side walls each have an inner and an outer surface, wherein the inner surface is suitable for contacting the liquid contents of the bag.

In some embodiments, the conduit 34 comprises a copolymer comprising ethylene and an acrylate. In those embodiments wherein the bag and the conduit both comprise a copolymer comprising ethylene and an acrylate, the bag and the conduit can include the same copolymer, or a different copolymer or a different combination of copolymers.

The conduit 34 can be placed in fluid communication with a filter device such as an in-line blood filter device (not shown) and/or can be placed in fluid communication with other containers (not shown), e.g., to provide a biological fluid processing system, that is preferably a closed system. The system can provide for pooling a plurality of units of biological fluid. In those embodiments wherein the biological processing system comprises an additional bag and conduit, typically, a plurality of bags and a plurality of conduits, the bags and conduits can be made from the copolymers as described above with respect to bag 30 and conduit 34, or they can be made from conventional polymers and/or copolymers as is known in the art.

In accordance with the invention, the containers and conduits comprising at least one copolymer comprising ethylene and an acrylate are suitable for a variety of medical, biomedical, and biotechnical applications. Containers and conduits produced according to the invention are typically flexible, and in some embodiments allow suitable gas transmission into and/or out of the interior volumes of the containers and conduits. For example, the polymeric film comprising the copolymer is flexible, permitting the film to be formed and sealed in a variety of shapes, and preferably has the quality of a suitable gas transmission for the desired application, e.g., a 22° C room air oxygen transmission of about 12 µmoles or greater O₂/hr/350 cm² film surface area. In some embodiments, the containers and/or conduits are also resilient to temperature fluctuations, e.g., they can withstand low temperatures during freezing, e.g., when processing plasma.

Typically, the containers and conduits produced in accordance with the invention are free of, or essentially free of, plasticizers such as di (2-ethylhexyl) phthalate (DEHP), tri (2-ethylhexyl) trimellitate (TOTM), and citrate ester plasticizers such as n-butryl tri-n-hexyl citrate (BTHC). However, the conduits and containers (e.g., the polymeric film) can include modifiers and/or additives such as, for example, at least one of an antistatic, antiblock, a stabilizer, and antioxidant, e.g., for use in processing the film or resin (described below).

Typically, a resin is used in producing the polymeric film (and in some embodiments, the conduit) and the resin comprises at least one copolymer comprising ethylene and an acrylate, preferably comprising ethylene and an alkyl acrylate. The resin can comprise a plurality of copolymers, e.g., a blend comprising a first copolymer comprising ethylene and a first alkyl acrylate, and a second copolymer comprising ethylene and a second alkyl acrylate.

In some embodiments, particularly embodiments of the container, the copolymer comprises ethylene and at least about 20 weight percent alkyl acrylate based upon the combined weight of the ethylene and the alkyl acrylate. For example, the copolymer can comprise ethylene and at least about 22 weight percent alkyl acrylate, or ethylene and at least about 24 weight percent alkyl acrylate. The term "alkyl" herein refers to an alkyl group having from 1 to about 10 carbon atoms, preferably from 1 to about 6 carbon atoms, and more preferably from 1 to about 4 carbon atoms. In even more preferred embodiments, the alkyl acrylate is methyl acrylate or butyl acrylate. For example, the resin can comprise a copolymer comprising ethylene, and at least about 20 wt. % methyl acrylate or at least about 20 wt. % butyl acrylate. In other embodiments, the resin comprises a copolymer comprising ethylene, and at least about 22 wt. % methyl acrylate or at least about 22 wt. % butyl acrylate, or ethylene and at least about 24 wt. % methyl acrylate or at least about 24 wt. % butyl acrylate.

Typically, the resin has a melt index of about 3 g or less per 10 min as measured by ASTM D 1238, condition 190°C/2.16 kg, and has a Vicat softening temperature (e.g., as measured by ASTM D 1525) of at least about 50°C.

Such resins are commercially available, e.g., from Eastman Chemical Company, Kingsport, TN. For example, a variety of resins commercially available from Eastman Chemical Company referred to as EMAC® (including EMAC+®), EBAC® (including EBAC+®), and EMAC/ EBAC® are suitable. Illustrative examples of such resins are ethylene butyl acrylate copolymer (EBAC) resin, e.g., EBAC SP1802 and SP1903 specialty copolymers, and ethylene methyl acrylate copolymer (EMAC) resin, e.g., EMAC SP1305, SP1307, SP1330, SP1400, SP2202, SP2207, SP2220, SP2260 and SP2268, specialty copolymers.

The bags and conduits according to the invention can have any suitable size, shape, internal volume and/or thickness. The bags and conduits can be made from the polymeric film and resin described herein using conventional techniques known and used in the industry. Illustratively, the bag can be arranged from a single sheet of sheet of film (e.g., folded over at the end where the ports are arranged and sealed around the other edges as shown in the Figure), two sheets of film, from a collapsed blown bubble of film (sometimes referred to as "lay flat tubing"), and the like. The bags and conduits are typically extruded, but can be blow molded or formed by other appropriate methods known in the art.

The preferred wall thickness of containers for biological fluids using the polymeric film can be in the conventional range of about 0.005 to about 0.025 inch (about 0.13 to about 0.64 mm), preferably about 0.010 inch to about 0.018 inch (about 0.25 to about 0.46 mm), with about 0.012 to about 0.015 inch (about 0.30 to about 0.38 mm) being most preferred. This wall thickness results in containers having sufficient tensile strength to withstand conventional use in the collection and processing of blood and blood components.

The walls can consist essentially of a polymeric film manufactured from at least one copolymer comprising ethylene and butyl acrylate or methyl acrylate.

In typical embodiments of containers according to the invention, each side wall is a single layer of film.

Preferably, the polymeric film has a 22° C room air oxygen transmission of about 12 µmoles or greater O₂/hr/350 cm² film surface area. In some embodiments, the 22° C room air oxygen transmission is 15 µmoles or greater O₂/hr/350 cm² film surface area, preferably, about 18 µmoles or greater O₂/hr/352 cm² film surface area, and even more preferably, about 20 µmoles or greater O₂/hr/350 cm² film surface area.

Containers and conduits can be sealed as is known in the art, utilizing, for example, an adhesive, a solvent, radio frequency sealing, ultrasonic sealing and/or heat sealing. If desired, at least one port (or fitment) is formed using the copolymer described above, and/or by co-extruding other materials such as various polymeric materials. For example, at least one port (or any number of ports) can have an outer surface material of the copolymer comprising ethylene and an acrylate, and an inner surface material of polyvinyl chloride (PVC). Such a configuration can allow efficient formation of the seal between outer surface of the port and the bag body, and efficient formation of the seal between the inner surface of the port with a conduit comprising PVC.

Containers according to the invention can have any suitable number of ports, and typically have at least two, and more preferably, at least three, ports. Ports can be suitable for accepting conduits (e.g., to allow connection to other system components), and/or suitable for spike entry. In one embodiment, the container has two ports suitable for accepting conduits, and two spike entry ports.

The inner and/or outer surfaces of the container side walls can be treated (e.g., to provide at least one of a coating, a chemical modification and a texture such as an embossment or etching) or the surfaces can be untreated.

Additionally, or alternatively, the container side walls can be formed by co-extruding various materials as described above with respect to the ports. Illustratively, a polymeric film can have one surface (e.g., the inner surface or the outer surface) material of the copolymer comprising ethylene and an acrylate, and another surface (e.g., the outer surface or the inner surface) material of another material such polyvinyl chloride (PVC). Such a configuration can provide one or more desired characteristics, e.g., the ability to withstand high temperatures. Typically, however, the inner and outer surfaces are formed from the same material, e.g., the side walls are each a single layer of a polymeric film manufactured from at least copolymer comprising ethylene and an acrylate.

The containers and conduits can be sterilized as is known in the art, e.g., via steam, ethylene oxide (ETOH), or gamma, sterilization.

In accordance with embodiments of a method according to the invention, a biological fluid, preferably a platelet-containing biological fluid (e.g., apheresis platelets, platelets obtained from platelet-rich-plasma or platelets obtained from pooled buffy coats), is passed into the container 30, and stored for a desired period of time before further use, e.g., as a transfusion product that is administered to a patient. Containers according to the invention are especially suitable for storing platelet-containing biological fluids that have been mixed with a platelet additive solution (PAS), e.g., wherein the PAS is utilized as a substitute for plasma. For example, a portion of the plasma in the biological fluid can be removed from a platelet-containing solution before storage, and the volume of the removed plasma can be replaced with or supplemented by the additive solution, more preferably wherein the additive solution comprises a protein-free medium. Since some volume of plasma remains with the platelets, and plasma includes protein, the addition of protein-free additive solution to the plasma-depleted platelets provides for platelet storage in a protein-poor solution. Preferably, the plasma-depleted platelets/additive solution mixture in the container 30 has, when compared to the original protein concentration (e.g., the protein concentration in the collected whole blood, or in the non-plasma depleted platelet-containing solution, wherein the original protein concentration is typically in the range of about 5.7 to about 6.4 g/dl), a residual protein concentration of about 35% or less, e.g., in the range of from about 10% to about 35%. In more preferred embodiments, the residual protein concentration is about 30% or less, and even more preferably, about 25% or less, compared to the original protein concentration. In some embodiments, the residual protein concentration is about 10%.

A variety of additive solutions are suitable for use according to the invention. For example, suitable platelet additive solutions are commercially available from Baxter Health Care (Deerfield, IL) under the tradenames PAS-I, PAS-II, PAS-III, and T-SOL®.

In accordance with current U.S. practice, platelet-containing biological fluids prepared in closed systems (with or without additive solutions) can be stored for 5 days before use, e.g., as transfusion products, and platelets stored in containers according to the invention can be stored for that period of time. However, studies of platelets stored in containers produced in accordance with embodiments of the invention show the platelets remain viable for longer periods of time, e.g., they remain viable after 7 days of storage, after 10 days of storage, and even after 14 days of storage. Accordingly, should the regulations in the U.S., or any other country be changed, embodiments of the invention allow for platelet storage for longer than 5 days, e.g., up to about 7 days or more, or 10 days, or even 14 days, or more.

The viability of the platelets can be determined by a variety of methods known in the art. Typically, in determining viability, at least one, and more preferably, two or more, of the following are evaluated: platelet count, pH, pO₂, pCO₂, bicarbonate, streaming (or swirling), hypotonic shock response (%HSR), extent of shape change (%ESC), % discs (platelet morphology), CD62 level (p-selectin), plasma glucose, and plasma lactate.

As noted above, embodiments of containers and/or conduits according to the invention can be utilized as part of a biological fluid processing system. In one preferred embodiment of a system according to the invention, the system includes a plurality of flexible bags, wherein at least one bag has at least one port and the top and at least one port at the bottom of the bag, and the bag is in fluid communication with at least one other bag, wherein the other bag has first and second side walls, the walls comprising a polymeric film manufactured from at least one copolymer comprising ethylene and an alkyl acrylate.

Embodiments of the biological fluid processing system according to the invention can include additional components, such as, for example, filter devices, including leukocyte depletion filter devices, as well as additional conduits, containers, one or more connectors, and one or more flow control devices such as clamps, transfer leg closures, and valves. Additionally, or alternatively, the system can include at least one of the following: a vent such as a gas collection and displacement arrangement, one or more gas inlets and/or one or more gas outlets.

The following examples further illustrate the invention but, of course, should not be construed as in any way limiting its scope.

### EXAMPLE 1

This example shows platelets stored in two embodiments of bags according to the invention maintain viability beyond a 5 day storage period.

A 300 mL bag is manufactured from an ethylene butyl acrylate copolymer (EBAC) resin, EBAC SP1802 (22.5 wt. % butyl acrylate comonomer; randomly distributed; melt index of 0.5 g/10 min as tested by ASTM D 1238, condition 190°C/2.16 kg; Vicat softening temperature of 60°C) specialty copolymer (Eastman Chemical Co., Kingsport, TN). Additionally, a 300 mL bag is manufactured from an ethylene methyl acrylate copolymer (EMAC) resin, EMAC SP2260 (24 wt. % methyl acrylate comonomer; randomly distributed; melt index of 2.1 g/10 min as tested by ASTM D 1238, condition 190°C/2.16 kg; Vicat softening temperature of 50°C) specialty copolymer (Eastman Chemical Co.)

The resins are processed to form single-layer polymeric films approximately 0.014 inches in thickness, having smooth inner surfaces. Two sheets of film are sealed together via radio frequency (RF) welding around the four edges and the fitments to form each bag, and the bags are sterilized via gamma sterilization.

Additionally, four other bags are obtained: two plasticized standard polyvinyl chloride (PVC) resin bags, a plasticized ultra-high-molecular weight PVC resin bag, and a bag prepared from ethylene vinyl acetate (EVA). One of the plasticized PVC bags is sterilized via steam, and the remaining three bags are sterilized via gamma sterilization.

Six units (450 mL) of anticoagulated whole blood are collected and each is processed to provide a unit of buffy coat (the buffy coat being a plasma-depleted platelet-containing fluid). The 6 units (50 mL each) of buffy coat are pooled and mixed with 600 mL of a commercially available protein-free additive solution (PAS-II, Baxter Health Care, Deerfield, IL), and the mixture is processed (including centrifugation to separate the platelets from the sedimented red cells and leukocytes, and passing the supernatant platelets through a leukocyte depletion filter) to prepare 300 mL of pooled buffy coat platelet concentrate in additive solution.

One hundred mL of the platelet concentrate (containing about 75 mL additive solution and about 25 mL plasma) is passed is passed into each of the 6 bags.

The bags are stored on a flatbed agitator set at 22°C. Samples of the platelets are taken from each bag and tested at days 1, 2, 5, 7, and 9 from the collection day. The following tests are carried out: platelet count, pH, pO₂, pCO₂, bicarbonate, streaming, hypotonic shock response (%HSR), extent of shape change (%ESC), % discs (platelet morphology), plasma glucose, and plasma lactate. In view of the results of the tests, the platelets maintain good viability for 9 days from the collection day in the EBAC, EMAC, and EVA bags, and the platelets do not maintain good viability for 9 days in the plasticized bags.

This example shows platelets stored in protein-poor solutions in a container produced from an ethylene butyl acrylate copolymer (EBAC) resin, and in a container produced from an ethylene methyl acrylate copolymer (EMAC) resin, maintain good viability when stored 9 days beyond the collection day, and this time period is beyond the current 5 day storage limit in accordance with U.S. regulations for stored platelets.

### EXAMPLE 2

Eight 1 Liter bags are manufactured from EBAC resin as generally described in Example 1, and eight plasticized standard polyvinyl chloride (PVC) resin bags are obtained. Each of the bags is sterilized via gamma sterilization.

Units of whole blood are processed to provide units of buffy coat, which are leukocyte-depleted, pooled, mixed with additive solution (T-SOL®, Baxter Health Care, Deerfield, IL), and separated to provide platelet concentrate in additive solution, and stored in individual bags as generally described in Example 1. Residual protein levels in the individual bags containing platelet concentrate, determined by the biurete method, range from about 0.96 g/dl to about 1.49 g/dl (about 17.4% to about 24.1% of the original protein concentration).

Samples of the platelets are taken from each bag and tested at days 1, 2, 5, 7, and 9 from the collection day. The following tests are carried out: platelet yield, pH, pO₂, pCO₂, swirling, %HSR, %ESC, % discs, plasma glucose, and plasma lactate.

In view of the results of the tests, the platelets maintain good viability for 9 days from the collection day in the EBAC bags, and the platelets do not maintain good viability for 9 days in the plasticized bags. In particular, platelets in the EBAC bags exhibit greater metabolic efficiency (e.g., an increased rate of oxygen consumption) than the platelets in the plasticized bags, and increased oxygen consumption results in reduced glucose consumption and lactate production.

This example shows platelets stored in a less than 25% residual protein concentration solution in an embodiment of a bag according to the invention maintain viability for a 9 day storage period.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Of course, variations of those preferred embodiments will become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

The present invention especially resides in the following embodiments:
1. A biological fluid container comprising:
   a container having an internal volume, the container having first and second side walls, the walls comprising a polymeric film manufactured from a copolymer comprising ethylene and an alkyl acrylate, the alkyl acrylate comprising butyl acrylate or methyl acrylate.
2. The container of embodiment 1, wherein the copolymer comprises ethylene and at least about 20 weight percent butyl acrylate or ethylene and at least about 20 weight percent methyl acrylate.
3. The container of embodiment 1, wherein the alkyl acrylate is butyl acrylate.
4. The container of embodiment 1, wherein the alkyl acrylate is methyl acrylate.
5. The container of embodiment 1, wherein the copolymer comprises ethylene and at least about 22 weight percent butyl acrylate or ethylene and at least about 22 weight percent methyl acrylate.
6. A biological fluid container comprising:
   a container having an internal volume, the container having first and second side walls, the walls comprising a polymeric film manufactured from at least one copolymer comprising ethylene and at least about 20 weight percent alkyl acrylate.
7. The container of embodiment 6, wherein the alkyl acrylate is methyl acrylate.
8. The container of embodiment 6, wherein the alkyl acrylate is butyl acrylate.
9. The container of any of embodiments 6-8, wherein the walls comprise a polymeric film manufactured from at least two copolymers comprising ethylene and an alkyl acrylate.
10. The container of any of embodiments 1-9, comprising a radio-frequency sealable container.
11. The container of any of embodiments 1-10, wherein the walls consist essentially of a polymeric film manufactured from a copolymer comprising ethylene and butyl acrylate or methyl acrylate.
12. A system for processing a biological fluid comprising:
   at least two flexible containers, each container having at least two ports and an internal volume;
   at least one flexible hollow conduit in fluid communication with the two containers;
      wherein at least one container has first and second side walls, the walls comprising a polymeric film manufactured from a copolymer comprising ethylene and an alkyl acrylate, the alkyl acrylate comprising butyl acrylate or methyl acrylate.
13. The system of embodiment 12, wherein the copolymer comprises ethylene and at least about 20 weight percent butyl acrylate or at least about 20 weight percent methyl acrylate.
14. The system of embodiment 12 or 13, wherein the system includes at least one flexible hollow conduit manufactured from at least one copolymer comprising ethylene and an acrylate.
15. The system of any of embodiments 12-14, comprising a closed system.
16. A conduit for use with a biological fluid comprising:
   a flexible hollow tubing manufactured from at least one copolymer comprising ethylene and an acrylate.
17. The conduit of embodiment 16, wherein the acrylate is an alkyl acrylate.
18. The conduit of embodiment 17, wherein the alkyl acrylate is butyl acrylate or methyl acrylate.
19. A method for processing a biological fluid comprising:
   passing a biological fluid into the container of any of embodiments 1-11; and
   storing the biological fluid in the container.
20. The method of embodiment 19, wherein the biological fluid comprises a plasma-depleted platelet-containing biological fluid mixed with platelet additive solution.
21. The method of embodiment 20, comprising storing the plasma-depleted platelet-containing biological fluid mixed with platelet additive solution for at least two days.
22. The method of embodiment 19, comprising storing the biological fluid for at least two days.
23. A method for processing a biological fluid comprising:
   obtaining a platelet-containing biological fluid;
   mixing the platelet-containing biological fluid with a platelet-additive solution to provide a platelet-containing platelet additive mixture; and
   passing a platelet- and additive-containing fluid into a container having first and second side walls, the walls comprising a polymeric film manufactured from a copolymer comprising ethylene and an alkyl acrylate, the alkyl acrylate comprising butyl acrylate or methyl acrylate.
24. The method of embodiment 23, including obtaining a platelet-containing biological fluid, and depleting plasma from the platelet-containing biological fluid to provide a plasma-depleted platelet-containing fluid; and
   mixing the plasma-depleted platelet-containing fluid with the platelet-additive solution to provide the platelet-containing platelet additive mixture.
25. The method of embodiment 24, wherein the residual protein concentration in the platelet- and additive-containing fluid in the container is about 35% or less of the protein concentration in the non-plasma-depleted platelet-containing biological fluid.
26. The method of any of embodiments 23-25, further comprising storing the platelet- and additive-containing fluid in the container for at least 2 days.
27. The method of embodiment 26, comprising storing the platelet- and additive-containing fluid in the container for at least 5 days.
28. The method of any of embodiments 20, 21, and 21-27, further comprising administering the platelets to a patient.

## Claims

1. A biological fluid container comprising:
a container having an internal volume, the container having first and second side walls, the walls comprising a polymeric film manufactured from a copolymer comprising ethylene and an alkyl acrylate, the alkyl acrylate comprising butyl acrylate or methyl acrylate.

2. The container of claim 1, wherein the copolymer comprises ethylene and at least about 20 weight percent butyl acrylate or ethylene and at least about 20 weight percent methyl acrylate.

3. The container of claim 1, wherein the copolymer comprises ethylene and at least about 22 weight percent butyl acrylate or ethylene and at least about 22 weight percent methyl acrylate.

4. A biological fluid container according to claim 1,
wherein said polymeric film is manufactured from at least one copolymer comprising ethylene and at least about 20 weight percent alkyl acrylate.

5. The container of claim 4, wherein the alkyl acrylate is methyl acrylate or butyl acrylate.

6. The container of claim 4 or 5, wherein the walls comprise a polymeric film manufactured from at least two copolymers comprising ethylene and an alkyl acrylate.

7. The container of any of claims 1 to 6, wherein the walls consist essentially of a polymeric film manufactured from a copolymer comprising ethylene and butyl acrylate or methyl acrylate.

8. A system for processing a biological fluid comprising:
at least two flexible containers, each container having at least two ports and an internal volume;
at least one flexible hollow conduit in fluid communication with the two containers;
wherein at least one container is a container according to any one of claims 1 to 7.

9. The system of claim 8 comprising a filter device, preferably a leukocyte depletion filter device.

10. Use of a container according to any one of claims 1 to 7 for storing a biological fluid.

11. The use of claim 10, wherein said biological fluid, wherein said biological fluid is blood, a blood component, or a blood product.

12. The use of claim 11, wherein said biological fluid is platelet concentrate, resuspended platelets, platelet-rich plasma, platelet-poor plasma, platelet-free plasma, plasma, or fresh frozen plasma.

13. A method for processing a biological fluid comprising:
passing a biological fluid into the container of any of claims 1 to 7; and
optionally storing the biological fluid in the container,
said biological fluid preferably comprising a plasma-depleted platelet-containing biological fluid mixed with platelet additive solution,
said method optionally comprising storing the plasma-depleted platelet-containing biological fluid mixed with platelet additive solution for at least two days,
or said method optionally comprising storing the biological fluid for at least two days.

14. A method for processing a biological fluid according to claim 13, comprising:
obtaining a platelet-containing biological fluid;
mixing the platelet-containing biological fluid with a platelet-additive solution to provide a platelet-containing platelet additive mixture; and
passing a platelet- and additive-containing fluid into a container having first and second side walls, the walls comprising a polymeric film manufactured from a copolymer comprising ethylene and an alkyl acrylate, the alkyl acrylate comprising butyl acrylate or methyl acrylate,
said method preferably including obtaining a platelet-containing biological fluid, and depleting plasma from the platelet-containing biological fluid to provide a plasma-depleted platelet-containing fluid; and
mixing the plasma-depleted platelet-containing fluid with the platelet-additive solution to provide the platelet-containing platelet additive mixture,
preferably wherein the residual protein concentration in the platelet- and additive-containing fluid in the container is about 35% or less of the protein concentration in the non-plasma-depleted platelet-containing biological fluid.

15. The method of claim 13 or 14, further comprising storing the platelet- and additive-containing fluid in the container for at least 2 days, preferably for at least 5 days.
